(19) **Europäisches Patentamt**

**European Patent Office**

**Office européen des brevets**

(11) **EP 1 046 037 B1**

(12) **FASCICULE DE BREVET EUROPEEN**

(45) Date de publication et mention de la délivrance du brevet:
**11.05.2005 Bulletin 2005/19**

(21) Numéro de dépôt: **99900498.9**

(22) Date de dépôt: **06.01.1999**

(51) Int Cl.[7]: **G01N 33/543**, H01F 1/11, B03C 1/01

(86) Numéro de dépôt international:
**PCT/FR1999/000011**

(87) Numéro de publication internationale:
**WO 1999/035500 (15.07.1999 Gazette 1999/28)**

(54) **PARTICULES MAGNETIQUES PERFECTIONNEES, LEURS PROCEDES D'OBTENTION ET LEURS UTILISATIONS DANS LA SEPARATION DE MOLECULES**

**PERFEKTIONIERTE MAGNETISCHE TEILCHEN, VERFAHREN ZU IHRER HERSTELLUNG UND IHRER ANWENDUNGEN ZUM TRENNEN VON MOLEKÜLEN**

**IMPROVED MAGNETIC PARTICLES, METHOD FOR OBTAINING SAME AND USES FOR SEPARATING MOLECULES**

(84) Etats contractants désignés:
**AT BE CH CY DE DK ES FI FR GB GR IE IT LI LU MC NL PT SE**

(30) Priorité: **06.01.1998 FR 9800220**

(43) Date de publication de la demande:
**25.10.2000 Bulletin 2000/43**

(73) Titulaire: **BIO MERIEUX**
**69280 Marcy l'Etoile (FR)**

(72) Inventeurs:
• **ELAISSARI, Abdelhamid**
**F-69007 Lyon (FR)**
• **PICHOT, Christian**
**F-69960 Corbas (FR)**
• **MANDRAND, Bernard**
**F-69100 Villeurbanne (FR)**

(74) Mandataire: **Guerre, Dominique et al**
**Cabinet Germain et Maureau,**
**12, rue Boileau,**
**BP 6153**
**69466 Lyon Cedex 06 (FR)**

(56) Documents cités:
**EP-A- 0 390 634        EP-A- 0 666 577**
**EP-A- 0 709 680        WO-A-94/09368**
**WO-A-97/45202**

• **KONDO A ET AL: "DEVELOPMENT AND APPLICATION OF THERMO-SENSITIVE MAGNETIC IMMUNOMICROSPHERES FOR ANTIBODY PURIFICATION" APPLIED MICROBIOLOGY AND BIOTECHNOLOGY, vol. 41, 1994, pages 99-105, XP000613881 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 046 037 B1

EP 1 046 037 B1

**Description**

La présente invention concerne des particules magnétiques et thermosensibles, ayant une taille prédéterminée comprise entre 0,05 et 10 μm, qui comprend, d'une part, un noyau composite interne et, d'autre part, une couche externe à base d'un polymère susceptible d'interagir avec au moins une molécule biologique. L'invention concerne également des procédés d'obtention et des utilisations de telles particules.

Dans l'exposé qui suit, le terme composite est synonyme de composé magnétique simple, tel que de la ferrite, ou complexe, tel que de la ferrite distribuée, incorporée ou enrobée dans une matrice polymère.

Ce noyau composite interne peut avoir un diamètre compris entre 20 nm et 10 μm.

Le diagnostic biologique consiste à déceler une maladie à partir de l'analyse de prélèvements biologiques (urines, sang, liquide céphalo-rachidien, sédiment, crachat, etc...). Dans ce cadre de la recherche, on souhaite notamment pouvoir extraire et concentrer, dans des conditions compatibles avec les tests d'activité de certaines protéines ou des acides nucléiques, afin de mettre en évidence la présence, chez les personnes malades, d'une protéine ou d'une séquence d'ADN ou d'ARN particulière. Dans le cas des protéines, les méthodes actuellement utilisées consistent à introduire dans le milieu une grande quantité de sels qui entraînent la précipitation des protéines, puis de séparer protéines et sels par centrifugation. Mais ces méthodes sont très lourdes et, en outre, les protéines ainsi extraites sont souvent dénaturées, c'est-à-dire qu'elles ont perdu leurs propriétés biologiques. Ceci explique l'intérêt porté, depuis quelques années, aux latex magnétiques dans le domaine biomédical.

En effet, les supports polymères sous forme de latex sont très utilisés car ils présentent de nombreux avantages, en particulier celui d'offrir une grande surface spécifique (plusieurs dizaines de m$^2$) par gramme de particule.

*L'état de la technique peut être défini par les documents EP-A-0.106.873 et EP-A-0.446.260 qui décrivent des particules superparamagnétiques et monodisperses comprenant un noyau poreux à base de copolymère polystyrène/divinylbenzène dans lequel sont incorporés des grains d'oxyde de fer magnétique, et une couche externe fonctionnalisée susceptible d'interagir avec des sondes d'acides nucléiques.*

Selon le procédé de préparation des particules décrites dans ces documents, les oxydes de fer magnétiques sont incorporés par précipitation des sels correspondants, ce qui limite la proportion de charge magnétique incorporée, et ne permet d'obtenir la charge magnétique qu'en une inonocouche en surface, ce qui peut induire des phénomènes d'inhibition des activités des biomolécules.

*De même, le document EP-A-0.585.868 décrit des particules magnétiques constituées par un noyau à base d'un premier polymère et d'une couche magnétique recouvrant le noyau constituée d'un second polymère dans lequel est distribué le matériau magnétique à base de ferrite, et capable d'interagir avec un antigène ou un anticorps, le matériau magnétique étant déposé par précipitation des sels de fer.*

Le matériau magnétique incorporé est directement exposé aux traitements ultérieurs des particules et il s'ensuit une perte de la charge au cours de l'utilisation des particules, ce qui peut entraîner des problèmes notamment d'inhibition enzymatique et de dénaturation d'entités biologiques.

*Le document WO-A-94/09368 a pour objet l'utilisation de gélatine en lieu et place du polymère à base de dérivés d'acrylamine, comme nous le préconisons. Ce document propose néanmoins un agent de réticulation.*

Pourtant la gélatine a des fonctions totalement différentes de celles d'un polymère tel que revendiqué. Ainsi la gélatine n'est absolument pas thermo-sensible et il n'était pas du tout avéré que la réticulation de dérivés d'acrylamine thermo-sensibles conduise à l'emprisonnement des inclusions magnétiques présentent dans le noyau interne.

*Selon l'article de A. KONDO, (A. KONDO, H. KAMURA, et K. HIGASHITANI (1994) Appl. Microbiol. Biotechnol., 41, 99-105) un procédé d'obtention de particules magnétiques est connu, qui comprend un noyau à base d'un premier polymère consistant en un polystyrène et dans lequel est distribué un matériau magnétique, et une couche hydrophile recouvrant le noyau, à base d'un polymère thermosensible consistant en du poly(N-isoproprylacrylamide). Le procédé décrit comprend les deux étapes suivantes. Selon une première étape pour l'obtention du noyau magnétique, on met en contact le matériau magnétique avec du styrène en présence d'un amorceur de polymérisation. Selon une seconde étape pour l'obtention de la couche hydrophile, on met en contact le noyau obtenu avec du N-isoproprylacrylamide et de l'acide méthacrylique, en présence de l'amorceur de polymérisation précédent. Sur les particules ainsi obtenues, on fixe de la sérumalbumine bovine pour ensuite procéder à l'isolement d'anticorps dirigés contre la sérum albumine bovine, présents dans un échantillon.*

Ce document propose des particules magnétiques qui comporte des inclusions de matière magnétique présente au niveau du noyau interne et/ou de la couche externe. Bien entendu, si ladite couche externe comporte des inclusions, celles-ci pourront être relarguées dans le milieu réactionnel et nuire à d'éventuelles applications et utilisations dans le domaine biologique et/ou du diagnostic nécessitant des réactions spécifiques. D'ailleurs, il n'y a pas d'utilisation d'agent de réticulation et donc aucune formation d'une couche interniédiaire. Si de telles particules sont mises en présence d'un solvant, les inclusions seront libérées dans le milieu réactionnel, même si ces inclusions ne sont présentes qu'au niveau du noyau interne. De plus, lesdites particules ne sont efficaces que pour les protéines. La séparation des acides nucléiques n'est pas envisageable.

*Ainsi pour assurer une séparation aussi efficiente que possible de ces particules dans l'échantillon, les demandeurs ont eu recours à une thermofloculation selon laquelle on augmente la température de l'échantillon, qui intervient pour compléter l'action d'un champ magnétique. Il s'agit du document WO-A-97/45202 qui propose des particules qui sont superparamagnétiques, qui ont une charge magnétique distribuée de manière très homogène, dont la proportion peut varier entre 1 et 80 %, en particulier de 25 et 80 % en poids par rapport au(x) polymère(s) constituant les particules. Ce document permet d'atteindre des proportions de charge magnétique incorporée élevées, en particulier car le procédé employé permet de répartir la charge magnétique en plusieurs niveaux. Il en résulte un avantage considérable à savoir la possibilité de séparer efficacement de l'échantillon, les particules de l'invention, sans avoir recours à l'action combinée d'une autre technique de séparation, telle que la floculation.*

Cette invention a un objectif différent de celui de la présente invention. De plus, il y a une couche intermédiaire magnétique qui recouvre un noyau interne qui n'est pas magnétique. On note par ailleurs l'absence de couche intermédiaire non magnétique. Cette configuration n'empêche pas le relargage des inclusions magnétiques de la couche intermédiaire dans le milieu réactionnel.

Si ces particules répondent à la plupart des attentes des biologistes, elles comportent un certain nombre d'inconvénients. Ainsi ces particules ne permettent pas, premièrement, de séparer les protéines des acides nucléiques, ce qui peut être particulièrement intéressant en fonction des travaux entrepris (identification d'anticorps, amplification de séquences, etc), et deuxièmement, les acides nucléiques. Ainsi, dans le cas de séparation d'acides nucléiques, les particules selon l'invention n'inhibent pas les différentes techniques d'amplification qui peuvent être utilisées (PCR, NASBA, TMA). Enfin, il n'est pas absolument nécessaire de relarguer les substances extraites (protéines ou acides nucléiques) par rapport aux particules, les tests d'identification ou d'amplification peuvent être effectués directement après la séparation magnétique.

A cet effet, la présente invention concerne des particules magnétiques et thermosensibles, ayant une taille prédéterminée comprise entre 0,05 et 10 $\mu$m, chaque paricule comprend :

- un noyau composite interne constitué d'une particule organique ou inorganique solide, contenant en son sein une charge magnétique, et
- une couche externe à base d'un polymère susceptible d'interagir avec au moins une molécule biologique, le polymère externe est thermosensible et présente une température critique inférieure de solubilité (LCST) prédéterminée comprise entre 10 et 100°C et de préférence entre 20 et 60°C,

caractérisée par le fait qu'une couche intermédiaire est présente entre le noyau interne et la couche externe, qui isole la charge magnétique dudit noyau interne par rapport à ladite couche externe fonctionnalisée.

Selon un mode préférentiel de réalisation, la couche intermédiaire est préparée par polymérisation d'au moins :

- un monomère fonctionnel ou non fonctionnel, pouvant polymériser afin de donner un polymère, et
- un agent de réticulation.

Selon un autre mode préférentiel de réalisation la couche externe est préparée par polymérisation d'au moins :

- un monomère fonctionnel, pouvant polymériser afin de donner un polymère, et
- un amorceur.

Selon une variante de réalisation, le monomère fonctionnel est constitué par un monomère cationique, tel que le chlorure de 2-aminoéthyl méthacrylate.

Selon une autre variante de réalisation, le monomère fonctionnel est constitué par un monomère anionique, tel qu'un monomère carboxylique ou sulfate.

Quel que soit le mode ou la variante de réalisation, le polymère est constitué de polymères hydrophiles, en particulier les dérivés acrylamines et de préférence le poly(NIPAM) obtenu par polymérisation de N-isopropylacrylamide (NIPAM).

Selon le premier mode préférentiel de réalisation, l'agent de réticulation est constitué par le N,N'-méthylène bisacrylamide.

Selon le second mode préférentiel de réalisation, l'amorceur est constitué par au moins un amorceur cationique, tel que le chlorure d'azobis (2-amidinopropane).

Toujours selon le second mode préférentiel de réalisation, l'amorceur est constitué par au moins un amorceur anionique, tel que le persulfate de potassium.

Dans tous les modes de réalisation précédemment exposés, il est possible que la couche externe soit fonctionnalisée par un groupe amine protoné ou amidine.

Dans tous les modes de réalisation précédemment exposés, il est possible que la couche externe soit fonctionnalisée par un groupe carboxylique ou sulfate.

La présente invention concerne également un procédé d'obtention de particules, telles que définies précédemment, qui consiste à mettre en présence et à faire réagir :

- un noyau composite constitué d'une particule organique ou inorganique solide, contenant en son sein une charge magnétique,
- un monomère fonctionnel ou non fonctionnel, pouvant polymériser afin de donner un polymère, et
- un agent de réticulation,

jusqu'à ce que le noyau composite, constituant le noyau interne, soit encapsulé et isolé par un mélange de polymère et d'agent de réticulation, et qu'une couche externe à base du polymère susceptible d'interagir avec au moins une molécule biologique soit formée.

Selon un mode préférentiel, les particules magnétiques cationiques sont réalisées par encapsulation par polymérisation, sur des noyaux composites, des monomères hydrosolubles d'acrylamide et/ou de dérivés d'acrylamide en présence du chlorure d'azobis (2-amidinopropane).

Avantageusement, la polymérisation s'effectue en présence de N,N'-méthylène bisacrylamide et/ou de chlorure de 2-aminoéthyl méthacrylate.

Selon un autre mode préférentiel, les particules magnétiques anioniques sont réalisées par encapsulation par polymérisation, sur des noyaux composites, des monomères hydrosolubles d'acrylamide ou de dérivés d'acrylamide en présence de persulfate de potassium.

Avantageusement, la polymérisation s'effectue en présence de N,N'-méthylène bisacrylamide et/ou de monomères carboxyliques.

Quel que soit le procédé mis en oeuvre, préalablement à la polymérisation, on recouvre les particules magnétiques, obtenues par adsorption ou polymérisation, par au moins une substance qui isole la charge magnétique, telle que du polystyrène et/ou du polyméthacrylate de méthyle.

Préférentiellement, le procédé consiste à utiliser les réactifs dans une proportion de :

- 10 à 30% de monomère hydrosoluble d'acrylamide ou d'un dérivé d'acrylamide,
- au plus 10% d'au moins un agent de réticulation, et
- 1 à 5% d'au moins un agent stabilisant,

par rapport à la masse totale de la particule obtenue par le procédé.

L'invention concerne encore l'utilisation de particules cationiques, telles que décrites ci-dessus, pour immobiliser ou extraire les acides nucléiques ou toutes entités polyélectrolytiques et pour relarguer et/ou analyser, après séparation magnétique, lesdits acides nucléiques ou entités, la température d'utilisation étant constamment inférieure à la LCST.

L'invention concerne enfin l'utilisation de particules anioniques, telles que décrites ci-dessus, pour extraire les protéines par fixation sur les particules lorsque la température est supérieure à la LCST et pour relarguer et/ou analyser, après séparation magnétique, lesdites protéines lorsque la température est inférieure à la LCST.

**I - Encapsulation des noyaux composites ou particules magnétiques commerciales :**

De nombreuses particules magnétiques sont commercialisées. La surface de ces particules est anionique ou cationique. En revanche, leur composition n'est jamais détaillée. Ces particules possèdent un indice de polymolécularité variable. En effet, les particules Seradyn, Spherotec et Dynal sont monodisperses alors que celles de chez Estapor, bien qu'ayant un diamètre moyen de 1 mm, sont très polydisperses.

Toutes ces particules ont été testées dans les procédés d'amplification d'acides nucléiques et elles engendrent des réactions d'inhibition. Il est donc nécessaire de modifier leur surface pour pouvoir travailler sur les acides nucléiques.

A/ Préparation du latex magnétique hydrophile cationique :

Les réactions d'encapsulation ont été réalisées sous atmosphère d'azote et à 70°C.

1 g de particules semence a été dilué avec 40 ml d'eau milliQ préalablement chauffée à ébullition et dégazée sous azote.

Le styrène a été ajouté simplement lorsque les particules semence sont hydrophobes. Il a été ajouté seul, avant le début de la synthèse, et laissé un certain temps, par exemple entre 30 et 60 minutes, en contact avec le noyau magnétique composite.

Les monomères ont été introduits dans les proportions suivantes:

- NIPAM: 0,3254 g soit 32,5% / semence (80%/polymère si la semence contient 60% de ferrite),
- MAB: 0,0274 g soit 6,18 mol%/NIPAM,
- AEM: 0,0740 g soit 15,5 mol%/NIPAM, et
- V50: 0,0061 g soit 0,80 mol%/NIPAM.

Il y a nécessité d'ajouter un tensioactif, par exemple, 0,14 g de triton X405.

L'amorceur est introduit avec 1 ml d'eau. Les monomères sont ajoutés par la suite. Ils sont dilués dans 9 ml et ils sont introduits progressivement dans le réacteur, c'est-à-dire dans un intervalle de temps de 15 minutes, ou ils sont introduits d'un seul coup dès l'ajout de l'amorceur.

Dans le cadre de la présente invention, des noyaux composites Estapor ont été encapsulées. Des particules anioniques (les particules EM1 100/20 et EM1 100/30) et des particules cationiques (les particules R95-07) ont été utilisées.

Les particules préparées à partir des latex EM1 100/20 et EM1 100/30 ont permis d'obtenir une densité de charge comprise entre 50 et 300 millimoles de $NH_2$ (mmol $NH_2$) par gramme de latex, suivant le mode de polymérisation utilisé (réacteur fermé, addition différé ou en continu).

Les particules préparées à partir du latex R95-07 ont permis d'obtenir une densité de charge comprise entre 50 et 300 mmol $NH_2$ par gramme de latex, suivant le mode de polymérisation.

Selon le protocole d'addition des monomères dans le système de réaction, on peut différencier plusieurs méthodes de polymérisation.

**1 - Polymérisation à réacteur fermé appelée « batch » :**

Les monomères sont introduits dans le réacteur avant le début de réaction avec les autres ingrédients et sans ajout ultérieur.

En raison de la différence de réactivité des monomères, ce procédé conduit souvent à l'apparition d'une dérive de composition. Celle-ci se manifeste par l'obtention de macromolécules ayant des compositions qui varient considérablement en fonction de la conversion. Cette méthode s'avère peu efficace pour l'incorporation en surface car une partie importante du monomère fonctionnel risque d'être perdue soit à l'intérieur des particules, soit sous forme de polymère hydrosoluble. Lorsque la copolymérisation est effectuée en « batch » avec des monomères de nature polaire, on obtient des particules plus petites, en grand nombre, mais avec une conversion limitée. Ce comportement est lié à l'importante solubilité dans l'eau de ces monomères, et il est attribué à la prépondérance du mécanisme de nucléation homogène.

**2 - Polymérisation en semi-continu :**

Une partie au moins des monomères est introduite dans le réacteur sur une période comprise entre le début de la réaction et la fin de celle-ci. Ce rajout peut être effectué à une vitesse fixe ou bien suivant un profil donné. Le but est de contrôler l'addition du mélange des monomères de façon à obtenir un copolymère de composition contrôlée ; c'est ainsi qu'on se place souvent dans des conditions d'addition telles que la vitesse de polymérisation soit plus grande que celle d'addition. Ceci permet d'obtenir des copolymères homogènes en composition.

**3 - Polymérisation par addition différée appelée « shot » :**

Lors d'une copolymérisation, et une fois que la réaction est en cours, le monomère fonctionnel seul, ou en présence du monomère de base, est introduit dans le système d'une façon contrôlée. Le succès de l'opération dépend donc du degré de connaissance préalable de la cinétique de copolymérisation. C'est une méthode efficace pour favoriser l'incorporation superficielle. La sélection des conditions expérimentales (degré de conversion au moment de l'addition, composition et concentration du mélange des monomères) permet d'optimiser les rendements de surface.

**4 - Polymérisation sur semence :**

Elle consiste à introduire le monomère fonctionnel dans le système contenant un latex déjà constitué et parfaitement caractérisé. Le monomère fonctionnel peut être additionné pur ou en mélange avec le monomère de base de la semence, en une étape ou en semi-continu.

Parmi les différentes techniques de polymérisation précitées, seules la polymérisation en réacteur fermé et la polymérisation sur semence ont été utilisées.

B/ Préparation du latex magnétique hydrophile anionique :

Les latex utilisés sont constitués d'un coeur de polystyrène et de ferrite, ce qui leur confère des propriétés magnétiques. Ils sont en outre recouverts, par polymérisation, de N-isopropylacrylamide (NIPAM). Un réticulant, le N, N'-méthylènebisacrylamide (MBA) est ajouté. L'amorceur utilisé est le persulfate de potassium ($K_2S_2O_8$), ce qui explique la présence de groupements sulfates en surface du latex, et donc son caractère anionique.

## II - Séparation des acides nucléiques :

Cette invention a pour finalité de mettre au point un nouveau modèle de test de diagnostic, qui devrait permettre de déceler de manière très précoce la présence de gènes infectieux chez un individu. Il s'agit d'établir un protocole permettant d'extraire spécifiquement des ADN ou des ARN de manière à pouvoir les doser tout en ayant au préalable éliminé les protéines présente en grande quantité dans le milieu de prélèvement. Le support choisi doit dans une première étape ne pas induire de réaction d'inhibition des procédés d'amplification des ADN et des ARN, de type PCR, NASBA ou TMA. Cette étape étant positive, le support devra être capable d'isoler spécifiquement des acides nucléiques en quantité suffisante.

Le support doit donc être hydrophile et ne pas relarguer de substances inhibitrices des réactions d'amplification.

Cette étude consiste à obtenir des latex magnétiques cationiques recouverts d'une chevelure hydrophile constituée de N-isopropylacrylamide (NIPAM) et fonctionnalisée par le chlorure de 2-aminoéthyl méthacrylate (AEM).

La surface des noyaux composites ou particules commerciales a été modifiée et les noyaux ou particules ont été encapsulées. Les principales particules disponibles sont les particules de Dynal, Seradyn, BioMag, Spherotec ou Estapor (marques déposées).

La demande de brevet FR96/04691, déposée le 9 avril 1996 par le demandeur, concerne une telle séparation des acides nucléiques. Son contenu est donc intégré à la présente demande de brevet.

## III - Séparation des protéines :

L'intérêt des latex est d'être non seulement thermosensible mais aussi magnétique, ce qui permet d'éviter les étapes de centrifugation. Un simple aimant de laboratoire permet de séparer les particules magnétiques du reste du milieu. Cette technique présente donc l'intérêt d'être plus rapide et plus facilement automatisable dans la perspective d'une éventuelle utilisation industrielle.

Dans un premier temps, il est nécessaire d'optimiser les conditions physico-chimiques permettant de concentrer des solutions contenant l'albumine humaine (HSA : Human Serum Albumine), protéine modèle. Dans un deuxième temps, il faut trouver les conditions qui permettent de concentrer l'ensemble des protéines présentes dans les urines.

Les tests sur des solutions préparées à partir de HSA seule ont été réalisés avec un coeur EM1 070/60 alors que les applications sur les urines ont été réalisées avec un coeur EM1 100/20.

Après lavage des noyaux composites à l'eau milliQ dégazée et bouillie sous azote pendant 1 heure, 1 g de latex estapor en solution dans 40 cm$^3$ d'eau est introduit dans le réacteur, maintenu pendant toute l'expérience à 70°C et sous courant d'azote. Après ajout de 200 ml de styrène, on laisse gonfler pendant 2 heures.

0,0061 g de KPS sont dissous dans 1 ml d'eau. Puis, toutes les minutes, on ajoute 1 ml d'une solution de 0,325 g de NIPAM, 0,027 g de MBA et 0,14 g de triton (X-405) dans 9 ml d'eau ; ce mélange est laissé à l'agitation pendant au moins 10 heures.

Il y a ensuite le lavage du latex.

Cette opération a pour but d'éliminer le tensioactif, ainsi que les électrolytes et les chaînes polymères libres en solution. En effet, le tensioactif peut entraîner des perturbations avec le réactif de Bradford (méthode chimique utilisée pour doser les protéines) et peut diminuer les capacités d'adsorption des latex.

Le latex utilisé étant magnétique, il est très facile, en utilisant un aimant, d'enlever le surnageant, de le remplacer par de l'eau milliQ fraîchement permutée, puis de l'agiter vigoureusement en utilisant un vortex. on recommence ce lavage jusqu'à ce que le surnageant soit limpide (on peut aussi vérifier que la conductivité du latex après le dernier lavage est pratiquement aussi faible que la conductivité de l'eau milliQ ($\approx$ 0,02 mS)).

A/ L'albumine du sérum humain (HSA) :

HSA est une protéine modèle pour étudier différents paramètres, tels que pH, force ionique, permettant de concentrer la protéine au maximum.

Il s'agit d'une protéine ellipsoïdale de dimensions approximatives $4 \times 4 \times 14$ nm et de poids moléculaire voisin de 62000 g/mol.

Son potentiel zéta (mesure physico-chimique relative à la densité de charge à la surface de la particule) varie avec le pH : la protéine HSA est cationique pour pH<PI (Point Isoélectrique : valeur du pH pour laquelle la charge nette est nulle ; PI=4,8 pour HSA) et anionique pour pH>PI.

B/ Méthodes de mesure de la concentration en protéines :

Le dosage des protéines, par exemple la HSA, est effectué en utilisant le réactif de Bradford. Ce dosage est possible grâce à l'utilisation du bleu de Coomassie (G 250) qui forme un complexe avec les protéines (initialement brun, il se colore en bleu d'autant plus vif que la concentration en protéines augmente). Le complexe protéine/colorant a un coefficient d'extinction très élevé, ce qui permet d'avoir une méthode très sensible. De plus, la coloration est quasi-instantanée, ce qui permet un dosage rapide.

Tous les dosages Bradford sont réalisés dans les mêmes conditions : 15 µl de la solution dont on veut déterminer la concentration en protéines

**+** 135 µl du tampon adéquat (pH et force ionique voulus)
**+** 150 µl de réactif de Coomassie.

**1 - Détermination de la quantité adsorbée :**

La quantité de protéines adsorbées Ns est donnée par la formule suivante :

$$Ns\ (mg.g^{-1}) = V\ (C_i\text{-}C_f)\ /\ m$$

avec :

$C_i$ (mg/ml) = concentration initiale en protéines de la solution en contact avec le latex ;
$C_f$ = concentration finale HSA dans le surnageant après adsorption ;
V (ml) = volume total de la solution ;
m (g) = masse de latex dans la solution.

**2 - Détermination de la quantité désorbée :**

Si les protéines étaient initialement adsorbées sur une masse m (en g) de latex et que, après désorption dans un volume V (ml), on détermine par dosage Bradford une concentration C (mg.ml$^{-1}$) de protéine, alors la quantité désorbée est donnée par :

$$Ns\ (mg.g^{-1}) = V*C\ /\ m$$

C/ Optimisation des paramètres :

**1 - Adsorption de la protéine HSA :**

Dans cette partie, on détermine les valeurs du pH, de la force ionique et de la température permettant d'adsorber sur les particules de latex le maximum de protéines.

**a. Mode opératoire :**
Les expériences sont réalisées dans des tubes eppendorf (contenance maximale : 1,5 ml).

| | |
|---|---|
| Volume de latex | 100 µl (soit 2,1 mg) |
| Volume de la solution de HSA | 200 µl (masse variable) |
| Volume de tampon | 700 µl |

Etant donné que l'on introduit 0,2 ml de solution de HSA de concentration donnée pour un volume total de 1 ml, la concentration réelle de la solution de HSA en contact avec le latex est obtenue après correction par un facteur de dilution de 0,2.

**b. Influence de la température :**

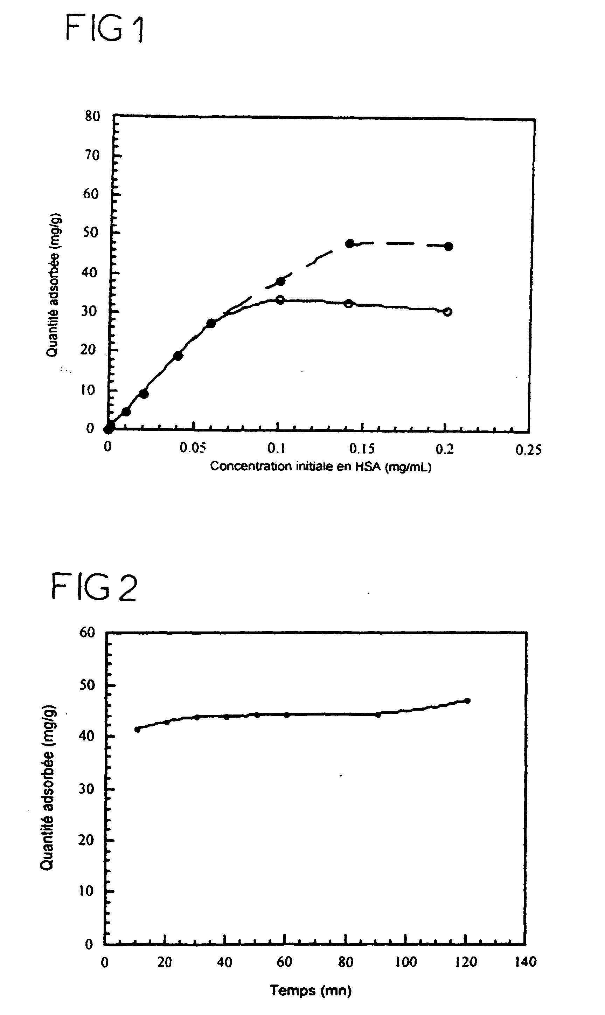

L'influence de la température sur l'adsorption est notable, indépendamment du pH. La figure 1 montre la variation de la quantité de protéines adsorbées en fonction de la concentration initiale en protéines, à deux températures différentes, pour un pH=4,7 et une force ionique de 0,01 mol/l.

La même tendance est observée à pH 5,7 (à 20°C, l'adsorption est 30 % plus faible qu'à 40°C) et à pH 8,6.

Puisque seule la nature hydrophile/hydrophobe du latex varie avec la température, cette étude permet de mettre en évidence l'influence des interactions hydrophobes sur l'adsorption.

Le fait que les courbes soient confondues pour les premiers points pourrait être dû au mode opératoire. Pour tester l'adsorption à 40°C, latex, protéines et tampon ont été mélangés à température ambiante puis chauffés jusqu'à 40°C. Il se peut donc que, pour les faibles concentrations en HSA, alors que nous pensions adsorber à 40°C, les protéines s'adsorbent sur le latex dans sa forme hydratée car T<32°C.

**c. Influence du temps d'adsorption :**

Dans les conditions jugées optimales pour l'adsorption, on réalise une étude cinétique de la quantité adsorbée en fonction du temps d'incubation. La figure 2 montre que, au bout de 30 minutes, on a déjà adsorbé pratiquement la quantité maximale possible.

**d. Influence du pH :**

L'adsorption a été réalisée pour les pH 4,7, 5,7 et 8,6 pour des concentrations en HSA comprises entre 0 et 0,2 $g.l^{-1}$. Pour l'adsorption à pH=8,6, on ne peut tracer la courbe pour (HSA)>0,06 $g.l^{-1}$ car la quantité adsorbée étant très faible, $(C_i - C_f)$ est quasiment nulle, et la méthode de mesure n'est pas assez précise pour détecter cette petite variation.

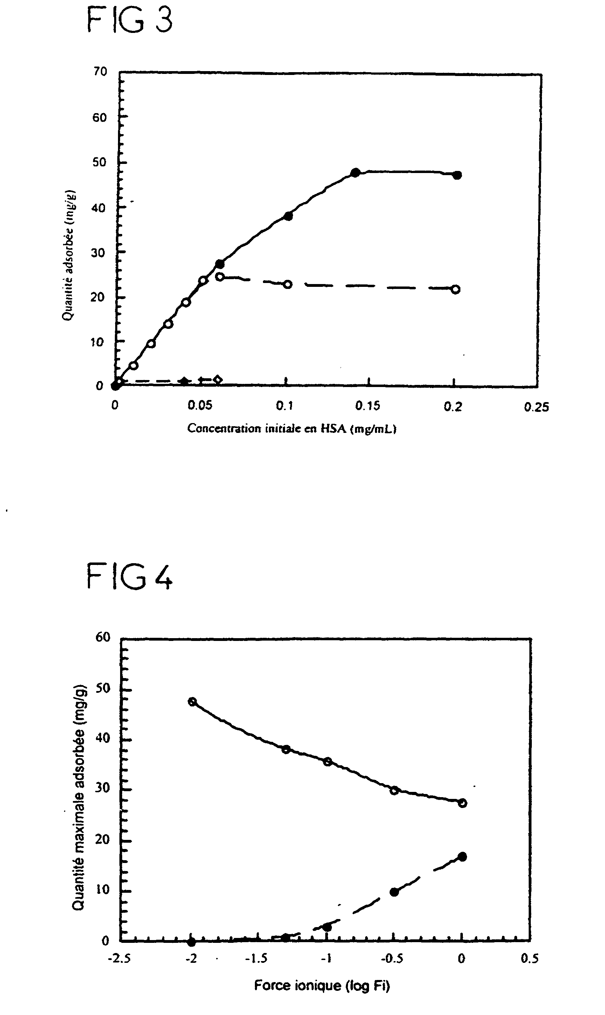

En superposant les trois courbes obtenues, on obtient le graphe de la figure 3 qui met en évidence :

i) l'existence d'un palier pour une adsorption de 50 mg de protéines par g de latex (saturation des sites d'adsorption) ;

ii) une adsorption maximale pour pH=4,7.

Ce résultat est en accord avec l'influence des interactions électrostatiques : pour pH<PI, la protéine est cationique et le latex anionique, d'où une *attraction* électrostatique et une adsorption favorisée, alors que pour pH>PI, la protéine et le latex sont anioniques, d'où une *répulsion* électrostatique qui réduit la quantité de HSA adsorbée. Cette étude met donc en évidence l'influence des forces électrostatiques sur l'adsorption.

**e. Influence de la force ionique :**

Les courbes donnant la quantité adsorbée en fonction de la force ionique, pour une même concentration initiale en protéine, sont reproduites sur la figure 4.

*i) pH=4,7 et (HSA)=0,14 $mg.ml^{-1}$ et T=40°C*

Cette courbe montre que l'adsorption est maximale pour une force ionique faible ; ceci est en accord avec des interactions électrostatiques attractives, qui favorisent d'autant plus l'adsorption qu'elles sont moins écrantées au fur et à mesure que la salinité du milieu diminue.

*ii) pH=8,6 et (HSA)=0,04 $mg.ml^{-1}$ et T=40°C*

Cette courbe montre que l'adsorption est maximale pour une force ionique élevée ; à pH basique, les interactions électrostatiques sont répulsives, donc l'adsorption est d'autant plus grande que ces interactions sont écrantées.

**f. Influence de la quantité de latex :**

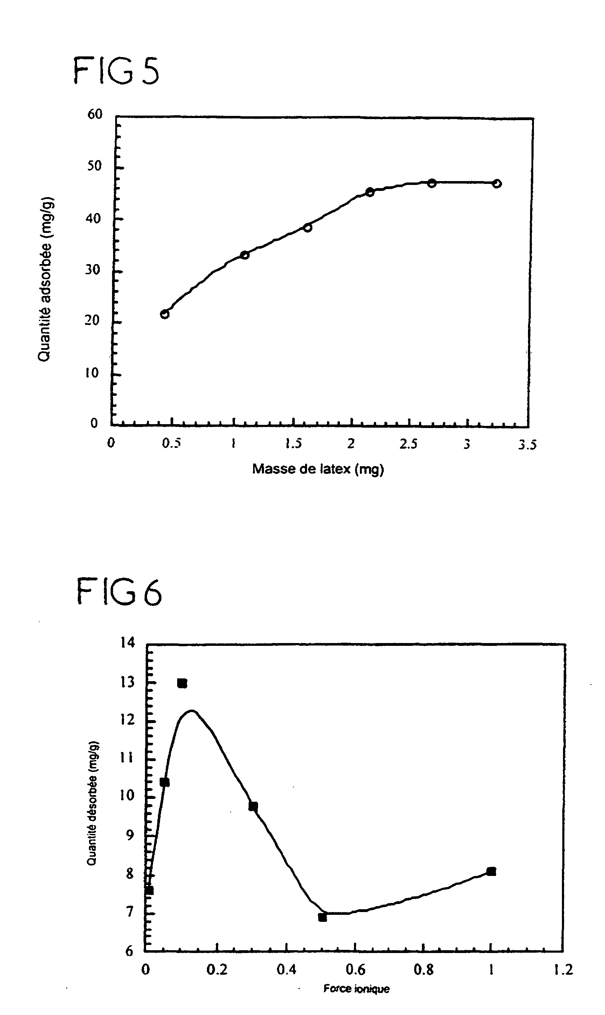

La figure 5 montre que, pour 0,14 mg de HSA et un latex de taux de solide 2,1 %, on atteint un palier de la quantité adsorbée pour un volume de 0,125 ml de latex (soit 2,6 mg). Ce palier correspond à approximativement 50 mg de protéines par gramme de latex soit, en supposant que les particules de latex ont un diamètre voisin de 1 mm et une densité de 1,85 $g/cm^3$ ($\Sigma$=3,24 $m^2/g$) : 15 mg de HSA par $m^2$ de polymère. Ce résultat est dans la moyenne attendue.

C'est donc ce rapport (environ 2 mg de latex pour 0,1 mg de protéines) qui sera utilisé par la suite.

**g. Bilan pour l'adsorption :**

Les paramètres optimisés pour l'adsorption sont donc :

pH=4,7

Fi=0,01 mol/l

T=40°C

t=10min

D/ Désorption de la protéine HSA :

L'adsorption est réalisée dans les conditions optimales déterminées ci-dessus, puis on étudie les paramètres qui gouvernent la désorption.

Les premiers tests de désorption ont été réalisés dans des tubes eppendorf. Tout d'abord, l'adsorption est conduite dans les conditions définies ci-dessus (on vérifie que la quasi-totalité des protéines présentes dans le milieu sont adsorbées). Le surnageant est ensuite soutiré puis remplacé par 1 ml de la solution dont on veut étudier la capacité à favoriser la désorption.

Après un temps de désorption variable, la mesure de la densité optique de ce nouveau surnageant permet de déduire la quantité de HSA désorbée, donc l'influence du temps de désorption.

La désorption se fait mieux à pH basique qu'à pH acide, et à 20°C mieux qu'à 40°C, ce qui était prévisible d'après l'influence du pH et de la température. En outre, on a choisi un temps d'adsorption faible ($\approx$ 10 min) car ce délai est suffisant pour adsorber la quasi-totalité des protéines, et que plus les protéines restent en contact avec le latex moins la désorption est importante.

L'influence d'autres paramètres était moins évidente, et les résultats obtenus sont détaillés ci-dessous.

**1 - Influence de la force ionique :**

La figure 6 montre l'influence de la force ionique du tampon sur la désorption.

Cette courbe fait apparaître la présence d'un maximum de désorption pour une force ionique proche de 0,1 mol.l$^{-1}$. Ce résultat est assez surprenant. A pH basique, le latex et HSA sont anioniques, donc les forces électrostatiques sont répulsives. Afin d'augmenter la désorption, il ne faut pas les écranter ; il faut a priori une force ionique faible. Mais, d'autre part, lorsque la force ionique augmente, la chevelure de polyNIPAM se trouve en mauvais solvant, donc se contracte, et les protéines se rapprochent. Comme elles portent la même charge négative, la répulsion électrostatique entraîne la désorption d'une certaine quantité de protéines. L'existence de deux phénomènes d'influences opposées justifie l'existence d'un maximum.

**2 - Influence du temps de désorption :**

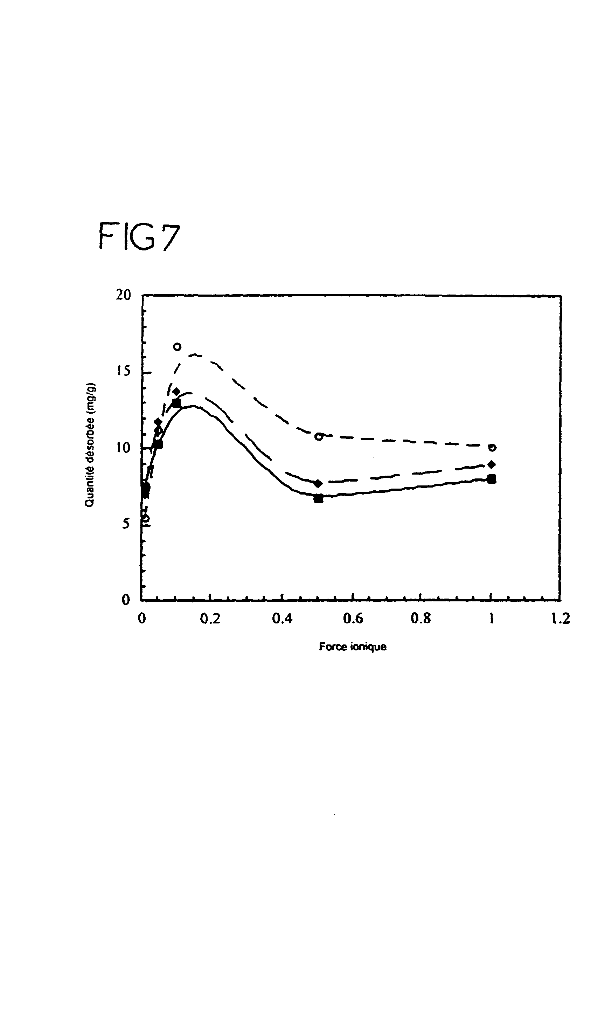

La figure 7 montre que la quantité désorbée augmente légèrement lorsque le temps de désorption augmente. Nous avons cependant choisi un temps de désorption assez faible (2h) pour des raisons pratiques : on désorbe ainsi environ 15% de moins que ce que l'on pourrait obtenir.

**3 - Bilan pour la désorption :**

Les paramètres optimisés pour la désorption sont donc :

pH>8,6

Fi=0,1 mol/l

T=20°C

t>2h

**E/ Bilan : concentration de solutions contenant de la HSA :**

L'adsorption est réalisée pour un volume total de 10 ml, dans les conditions optimales, dans des tubes "Falcon". Pour cela, après préparation d'une solution de HSA, dans un tampon de pH=4,7 et- de force ionique 0,01 mol/L, de concentration telle que la concentration de protéines réellement en contact avec le latex soit de 0,1 g.l$^{-1}$ compte tenu de la dilution, on introduit 27 mg de latex.

La quantité adsorbée est environ 0,095 mg.ml$^{-1}$ (soit 95% des protéines initialement présentes). Après séparation par utilisation d'un aimant, la désorption est ensuite réalisée en introduisant 1 ml de tampon de pH=8,6 puis pH=10,8. Dans le premier cas, la solution obtenue a une concentration en HSA égale à 0,23 mg.ml$^{-1}$ (on désorbe 24% de la quantité adsorbée) et 0,37 mg.ml$^{-1}$ (on désorbe 39%) dans le second. La concentration initiale peut donc, dans ces conditions, être multipliée au maximum par 3,7.

## IV - APPLICATION AUX URINES :

### A/ Remarque générale :

Les études préliminaires ont été effectuées sur un milieu extrêmement simple : une seule protéine, de concentration connue, dans un milieu tamponné dont tous les paramètres ont été déterminés.

En revanche, les urines constituent un milieu très complexe, contenant plusieurs dizaines de protéines de poids moléculaires différents, et riche en sels. Cependant, pour établir la concentration en protéines des urines à partir de la densité optique, nous avons utilisé les courbes d'étalonnage établies pour HSA : les résultats obtenus ne sont donc que qualitatifs. De plus, le pH et la concentration en protéines des urines dépend des personnes et de leur alimentation. Il est donc nécessaire de déterminer ces valeurs pour chaque lot d'urine utilisé.

### B/ Résultats pour des urines de témoins négatifs :

La première série d'expériences a été réalisée en utilisant les conditions des tests préliminaires. Dans la deuxième série, on a modifié certaines étapes en fonction des résultats de la première série.

**1 - Mode opératoire :**

Après introduction dans des tubes "Falcon" de 10 ml des urines à concentrer et de 27 mg de latex, le mélange est placé au bain-marie à 40°C pendant un temps variable. Ensuite, le surnageant est soutiré, puis remplacé par 1 mL de tampon à pH=4,7, que l'on enlève presque instantanément : cette opération a pour but de laver le latex, sans, en principe, désorber.

Après ajout de 1 mL de tampon de pH=10,6 et force ionique 0,01M, on laisse désorber à 20°C pendant un temps $t_D$. Le dosage Bradford du surnageant permet de déterminer la concentration totale en protéines désorbées.

**2 - Dosage des protéines (par Bradford) :**

Le pH des urines étudiées est voisin de 7 et la concentration initiale en protéines (pour le lot utilisé) est de l'ordre de 0,02 g.l$^{-1}$. L'analyse des surnageants montre que :

1) il est nécessaire d'acidifier les urines ; à leur pH naturel proche de 7, l'adsorption n'atteint pas 50% des protéines présentes, alors qu'elle approche les 80% dans les urines acidifiées à pH=4,6 ;

2) l'étape de lavage entraîne la perte d'environ 20% des protéines adsorbées ; nous avons donc supprimer cette étape dans les expériences suivantes.

L'étude de l'influence du temps d'adsorption et de désorption montre que, contrairement aux résultats obtenus lors de l'étude préliminaire sur HSA, il est nécessaire d'adsorber pendant au moins 60 mn pour atteindre le palier d'adsorption. Après un temps de désorption de 60 mn dans un volume de 1 ml, la solution obtenue est 4 fois plus concentrée en protéines que les urines de départ. Ce résultat est assez satisfaisant puisqu'il est en accord avec les tests de concentration de HSA seule, même si, en théorie, on pouvait espérer multiplier- la concentration par 10 puisque l'on concentre 10 ml en 1 ml.

Cependant, le but final n'est pas de concentrer toutes les protéines, mais d'isoler la protéine qui nous intéresse. D'où l'intérêt d'analyser les différents échantillons ainsi obtenus sur gel d'électrophorèse, pour mettre en évidence la ou les différentes protéines.

### V - CONCLUSIONS :

Une étude préliminaire a permis de vérifier que l'utilisation de particules de latex magnétiques et thermosensibles permet, en jouant sur différents paramètres physiques (pH, température, force ionique), d'adsorber et de désorber l'albumine humaine (HSA). Il est ainsi possible, à partir d'une solution de 10 ml diluée de cette protéine, de passer à une solution de 1 ml environ 4 fois plus concentrée.

En optimisant les paramètres, il est possible d'adsorber jusqu'à 95% des protéines. Le fait que l'on ne puisse concentrer plus est dû à une désorption incomplète. Afin d'augmenter ce taux de désorption, il est envisageable d'ajouter des tensioactifs susceptibles de s'échanger avec les protéines adsorbées.

L'application des conditions de concentration optimales pour HSA aux urines permet d'obtenir des résultats semblables. Il est possible de multiplier par quatre la concentration en protéines d'un lot d'urines en désorbant dans un tampon approprié. En lyophilisant les échantillons ainsi concentrés, on peut obtenir un facteur multiplicatif de 100, proche du taux de concentration obtenu par la méthode de précipitation au sulfate d'ammonium. Cependant, la solution obtenue après lyophilisation est trop concentrée en sels. Une étape de dialyse serait donc nécessaire afin de remplacer le tampon de force ionique 0,1 mol/l par un milieu moins salin, à moins que l'on ne décide de désorber dans un milieu de force ionique presque nulle, quitte à ne désorber qu'une plus faible quantité de protéines.

### Revendications

1. Particule magnétique et thermosensible, ayant une taille prédéterminée comprise entre 0,05 et 10 µm, qui comprend :

   - un noyau composite interne constitué d'une particule organique ou inorganique solide, contenant en son sein une charge magnétique, et
   - une couche externe à base d'un polymère susceptible d'interagir avec au moins une molécule biologique, le polymère externe est thermosensible et présente une température critique inférieure de solubilité (LCST) prédéterminée comprise entre 10 et 100°C et de préférence entre 20 et 60°C,

   **caractérisée par le fait qu'**une couche intermédiaire est présente entre le noyau interne et la couche externe, qui isole la charge magnétique dudit noyau interne par rapport à ladite couche externe fonctionnalisée et qu'elle est constituée par un polymère réticulé préparé à partir d'au moins un monomère fonctionnel ou non fonctionnel, pouvant polymériser afin de donner un polymère, et un agent de réticulation.

2. Particule, selon la revendication 1,
   **caractérisée par le fait que** la couche externe est preparée par polymérisation d'au moins :

   - un monomère fonctionnel pouvant polymériser afin d'obtenir un polymère, et
   - un amorceur.

3. Particule, selon la revendication 1 ou 2,
   **caractérisée par le fait que** le monomère fonctionnel est constitué par un monomère cationique, tel que le chlorure de 2-aminoéthyl méthacrylate.

4. Particule, selon la revendication 1 ou 2,
   **caractérisée par le fait que** le monomère fonctionnel est constitué par un monomère anionique, tel qu'un monomère carboxylique ou sulfate.

5. Particule, selon l'une quelconque des revendications 1 à 4, **caractérisée par le fait que** le polymère est constitué de polymères hydrophiles, en particulier les dérivés acrylamines et de préférence le poly(NIPAM) obtenu par polymérisation de N-isopropylacrylamide (NIPAM).

6. Particule, selon la revendication 1,
   **caractérisée par le fait que** l'agent de réticulation est constitué par le N,N'-méthylène bisacrylamide.

**7.** Particule, selon la revendication 2,
**caractérisée par le fait que** l'amorceur est constitué par au moins un amorceur cationique, tel que le chlorure d'azobis (2-amidinopropane).

**8.** Particule, selon la revendication 2,
**caractérisée par le fait que** l'amorceur est constitué par au moins un amorceur anionique, tel que le persulfate de potassium.

**9.** Particule, selon l'une quelconque des revendications 1 à 8, **caractérisée par le fait que** la couche externe est fonctionnalisée par un groupe amine protoné ou amidine.

**10.** Particule, selon l'une quelconque des revendications 1 à 9, **caractérisée par le fait que** la couche externe est fonctionnalisée par un groupe carboxylique ou sulfate.

**11.** Procédé d'obtention de particules, telles que définies selon l'une quelconque des revendications 1 à 10, **caractérisé en ce qu'**il consiste à :

- faire réagir un noyau composite constitué d'une particule organique ou inorganique solide, contenant en son sein une charge magnétique, et un monomère fonctionnel ou non fonctionnel pouvant polymériser, afin d'obtenir un polymère constituant une couche intermédiaire autour du noyau composite interne, et
- faire réagir un agent de réticulation pour réticuler le polymère, et
- faire réagir la couche intermédiaire polymérisée et réticulée avec un monomère fonctionnel pouvant polymériser, afin d'obtenir un polymère constituant une couche externe.

**12.** Procédé d'obtention de particules selon la revendication 11, **caractérisé en ce que** des particules magnétiques cationiques sont réalisées par polymérisation, de monomères hydrosolubles d'acrylamide et/ou de dérivés d'acrylamide en présence du chlorure d'azobis (2-amidinopropane).

**13.** Procédé selon la revendication 12,
**caractérisé en ce que** la polymérisation s'effectue en présence de N,N'-méthylène bisacrylamide et/ou de chlorure de 2-aminoéthyl méthacrylate.

**14.** Procédé d'obtention de particules selon la revendication 11, **caractérisé en ce que** des particules magnétiques anioniques sont réalisées par polymérisation de monomères hydrosolubles d'acrylamide ou de dérivés d'acrylamide en présence de persulfate de potassium.

**15.** Procédé selon la revendication 14,
**caractérisé en ce que** la polymérisation s'effectue en présence de N,N'-méthylène bisacrylamide et/ou de monomères carboxyliques.

**16.** Procédé selon l'une quelconque des revendications 11 à 15, **caractérisé en ce que** la couche intermédiaire est constituée par au moins une substance qui isole la charge magnétique, substance telle que du polystyrène et/ou du polyméthacrylate de méthyle.

**17.** Procédé selon l'une quelconque des revendications 11 à 15, **caractérisé en ce qu'**il consiste à utiliser les réactifs dans une proportion de :

- 10 à 30% de monomère hydrosoluble d'acrylamide ou d'un dérivé d'acrylamide,
- au plus 10% d'au moins un agent de réticulation, et
- 1 à 5% d'au moins un agent stabilisant,

par rapport à la masse totale de la particule obtenue par le procédé.

**18.** Utilisation de particules cationiques selon l'une quelconque des revendications 1 à 3, 5 à 7 ou 9, pour immobiliser ou extraire les acides nucléiques ou toutes entités polyélectrolytiques et pour relarguer et/ou analyser, après séparation magnétique, lesdits acides nucléiques ou entités, la température d'utilisation étant constamment inférieure à la LCST.

**19.** Utilisation de particules anioniques selon l'une quelconque des revendications 1 à 2, 4 à 6, 8 ou 10, pour extraire les protéines par fixation sur les particules lorsque la température est supérieure à la LCST et pour relarguer et/ ou analyser, après séparation magnétique, lesdites protéines lorsque la température est inférieure à la LCST.

**Patentansprüche**

**1.** Wärmeempfindliches magnetisches Teilchen mit einer vorbestimmten Größe zwischen 0,05 und 10 μm, enthaltend:

- einen inneren Verbundkern, der aus einem festen organischen oder anorganischen Teilchen besteht, das in seinem Inneren eine magnetische Ladung enthält, und
- eine äußere Schicht auf Basis eines zur Wechselwirkung mit mindestens einem biologischen Molekül befähigten Polymer, wobei das äußere Polymer wärmeempfindlich ist und eine vorbestimmte untere kritische Löslichkeitstemperatur (LCST) zwischen 10 und 100°C und vorzugsweise zwischen 20 und 60°C aufweist,

**dadurch gekennzeichnet, daß** sich zwischen dem inneren Kern und der äußeren Schicht eine Zwischenschicht befindet, die die magnetische Ladung des inneren Kerns gegenüber der funktionalisierten äußeren Schicht isoliert und aus einem aus mindestens einem zu einem Polymer polymerisierbaren funktionellen oder nichtfunktionellen Monomer, und einem Vernetzer hergestellten vernetzten Polymer besteht.

**2.** Teilchen nach Anspruch 1, **dadurch gekennzeichnet, daß** die äußere Schicht durch Polymerisation von mindestens

- einem zu einem Polymer polymerisierbaren funktionellen Monomer und
- einem Inititator

hergestellt wird.

**3.** Teilchen nach Anspruch 1 oder 2, **dadurch gekennzeichnet, daß** das funktionelle Monomer aus einem kationischen Monomer, wie 2-Aminomethylmethacrylatchlorid, besteht.

**4.** Teilchen nach Anspruch 1 oder 2, **dadurch gekennzeichnet, daß** das funktionelle Monomer aus einem anionischen Monomer, wie einem Carbonsäure- oder Sulfatmonomer, besteht.

**5.** Teilchen nach einem der Ansprüche 1 bis 4, **dadurch gekennzeichnet, daß** das Polymer aus hydrophilen Polymeren, insbesondere Acrylaminderivaten und vorzugsweise durch Polymerisation von N-Isopropylacrylamid (NIPAM) erhaltenem Poly(NIPAM), besteht.

**6.** Teilchen nach Anspruch 1, **dadurch gekennzeichnet, daß** der Vernetzer aus N,N'-Methylenbisacrylamid besteht.

**7.** Teilchen nach Anspruch 2, **dadurch gekennzeichnet, daß** der Initiator aus mindestens einem kationischen Initiator, wie Azobis(2-amidinopropan)chlorid, besteht.

**8.** Teilchen nach Anspruch 2, **dadurch gekennzeichnet, daß** der Initiator aus mindestens einem anionischen Initiator, wie Kaliumpersulfat, besteht.

**9.** Teilchen nach einem der Ansprüche 1 bis 8, **dadurch gekennzeichnet, daß** die äußere Schicht mit einer protonierten Amin- oder Amidingruppe substituiert ist.

**10.** Teilchen nach einem der Ansprüche 1 bis 9, **dadurch gekennzeichnet, daß** die äußere Schicht mit einer Carbonsäure- oder Sulfatgruppe substituiert ist.

**11.** Verfahren zur Herstellung von Teilchen gemäß einem der Ansprüche 1 bis 10, **dadurch gekennzeichnet, daß** man:

- einen Verbundkern, der aus einem festen organischen oder anorganischen Teilchen besteht, das in seinem Inneren eine magnetische Ladung enthält, und ein zu einem Polymer polymerisierbares funktionelles oder nichtfunktionelles Monomer zur Reaktion bringt, wobei man ein eine Zwischenschicht um den inneren Verbundkern herum bildendes Polymer erhält,

- zur Vernetzung des Polymers einen Vernetzer zur Reaktion bringt und
- die vernetzte polymerisierte Zwischenschicht mit einem polymerisierbaren Monomer zu einem eine äußere Schicht bildenden Polymer umsetzt.

**12.** Verfahren zur Herstellung von Teilchen nach Anspruch 11, **dadurch gekennzeichnet, daß** man durch Polymerisation von wasserlöslichen Acrylamidund/oder Acrylamidderivat-Monomeren in Gegenwart von Azobis(2-amidinopropan)chlorid kationische magnetische Teilchen herstellt.

**13.** Verfahren nach Anspruch 12, **dadurch gekennzeichnet, daß** man die Polymerisation in Gegenwart von N,N'-Methylenbisacrylamid und/oder 2-Aminomethylmethacrylatchlorid durchführt.

**14.** Verfahren zur Herstellung von Teilchen nach Anspruch 11, **dadurch gekennzeichnet, daß** man durch Polymerisation von wasserlöslichen Acrylamid- und/oder Acrylamidderivat-Monomeren in Gegenwart von Kaliumpersulfat anionische magnetische Teilchen herstellt.

**15.** Verfahren nach Anspruch 14, **dadurch gekennzeichnet, daß** man die Polymerisation in Gegenwart von N,N'-Methylenbisacrylamid und/oder Carbonsäuremonomeren durchführt.

**16.** Verfahren nach einem der Ansprüche 11 bis 15,
**dadurch gekennzeichnet, daß** die Zwischenschicht aus mindestens einer Substanz, die die magnetische Ladung isoliert, wie Polystyrol und/oder Polymethylmethacrylat, besteht.

**17.** Verfahren nach einem der Ansprüche 11 bis 15,
**dadurch gekennzeichnet, daß** man dabei die Reagenzien in einem Anteil von:

- 10 bis 30% wasserlöslichem Acrylamid- oder Acrylamidderivat-Monomer,
- höchstens 10% mindestens eines Vernetzers und
- 1 bis 5% mindestens eines Stabilisators,

bezogen auf die Gesamtmasse des nach dem Verfahren erhaltenen Teilchens, verwendet.

**18.** Verwendung von kationischen Teilchen gemäß einem der Ansprüche 1 bis 3, 5 bis 7 oder 9 zur Immobilisierung oder Extraktion von Nucleinsäuren oder beliebigen Polyelektrolyten und zur Freigabe und/oder Analyse der Nucleinsäuren oder Polyelektrolyte nach magnetischer Abtrennung, wobei die Verwendungstemperatur ständig unter der LCST liegt.

**19.** Verwendung von anionischen Teilchen gemäß einem der Ansprüche 1 bis 2, 4 bis 6, 8 oder 10 zur Extraktion von Proteinen durch Fixierung an den Teilchen bei einer über der LCST liegenden Temperatur und zur Freigabe und/oder Analyse dieser Proteine nach magnetischer Abtrennung bei einer unter der LCST liegenden Temperatur.

**Claims**

**1.** Heat-sensitive magnetic particle having a predetermined size between 0.05 and 10 µm, which comprises:

- an internal composite core which consists of a solid organic or inorganic particle, and which contains within itself a magnetic filler, and
- an external layer based on a polymer which is capable of interacting with at least one biological molecule, the external polymer is heat-sensitive and has a predetermined lower critical solubility temperature (LCST) between 10 and 100°C, and preferably between 20 and 60°C,

**characterized in that** an intermediate layer is present between the internal core and the external layer, which isolates the magnetic filler of said internal core with respect to said functionalized external layer and **in that** it consists of a crosslinked polymer prepared from at least one functional or nonfunctional monomer, which is able to polymerize in order to give a polymer, and one crosslinking agent.

**2.** Particle according to Claim 1, **characterized in that** the external layer is prepared by polymerization of at least:

- one functional monomer, which is able to polymerize in order to give a polymer, and
- one initiator.

3. Particle according to Claim 1 or 2,
   **characterized in that** the functional monomer consists of a cationic monomer such as 2 aminoethyl methacrylate chloride.

4. Particle according to Claim 1 or 2,
   **characterized in that** the functional monomer consists of an anionic monomer such as a carboxylic or sulphate monomer.

5. Particle according to any one of Claims 1 to 4,
   **characterized in that** the polymer consists of hydrophilic polymers, in particular the acrylamine derivatives, and preferably poly(NIPAM) obtained by polymerization of N isopropylacrylamide (NIPAM).

6. Particle according to Claim 1, **characterized in that** the crosslinking agent consists of N,N' methylenebisacrylamide.

7. Particle according to Claim 2, **characterized in that** the initiator consists of at least one cationic initiator such as azobis(2 amidinopropane) chloride.

8. Particle according to Claim 2, **characterized in that** the initiator consists of at least one anionic initiator such as potassium persulphate.

9. Particle according to any one of Claims 1 to 8,
   **characterized in that** the external layer is functionalized with a protonated amine or amidine group.

10. Particle according to any one of Claims 1 to 9,
    **characterized in that** the external layer is functionalized with a carboxylic or sulphate group.

11. Method for obtaining particles as defined according to any one of Claims 1 to 10, **characterized in that** it consists in:

    - reacting a composite core which consists of a solid organic or inorganic particle, and which contains within itself a magnetic filler, and a functional or nonfunctional monomer which is able to polymerize in order to obtain a polymer constituting an intermediate layer around the internal composite core, and
    - reacting a crosslinking agent to crosslink the polymer, and
    - reacting the polymerized crosslinked intermediate layer with a functional monomer which is able to polymerize in order to obtain a polymer constituting an external layer.

12. Method for obtaining particles according to Claim 11, **characterized in that** cationic magnetic particles are prepared by polymerization of water-soluble monomers of acrylamide and/or of acrylamide derivatives in the presence of azobis (2 amidinopropane) chloride.

13. Method according to Claim 12, **characterized in that** the polymerization is performed in the presence of N,N'-methylenebisacrylamide and/or of 2-aminoethyl methacrylate chloride.

14. Method for obtaining particles according to Claim 11, **characterized in that** anionic magnetic particles are prepared by polymerization of water-soluble monomers of acrylamide or of acrylamide derivatives in the presence of potassium persulphate.

15. Method according to Claim 14, **characterized in that** the polymerization is performed in the presence of N,N'-methylenebisacrylamide and/or of carboxylic monomers.

16. Method according to any one of Claims 11 to 15,
    **characterized in that** the intermediate layer consists of at least one substance which isolates the magnetic filler, this substance being for example polystyrene and/or methyl polymethacrylate.

17. Method according to any one of Claims 11 to 15,

**<u>characterized in that</u>** it consists in using the reagents in a proportion of:

- 10 to 30% of water-soluble monomer of acrylamide or of an acrylamide derivative,
- at most 10% of at least one crosslinking agent, and
- 1 to 5% of at least one stabilizer,

with respect to the total mass of the particle obtained by the method.

18. Use of cationic particles according to any one of Claims 1 to 3, 5 to 7 or 9, to immobilize or extract nucleic acids or any polyelectrolytic entities, and to release and/or analyse, after magnetic separation, said nucleic acids or entities, the temperature of use being constantly lower than the LCST.

19. Use of anionic particles according to any one of Claims 1, 2, 4 to 6, 8 or 10, to extract proteins by binding to the particles when the temperature is higher than the LCST, and to release and/or analyse, after magnetic separation, said proteins when the temperature is lower than the LCST.

FIG 1

FIG 2

## FIG 3

## FIG 4

# FIG 5

# FIG 6

FIG 7